# EUROPEAN PATENT SPECIFICATION

(11) **EP 3 488 867 B1**
(45) Date of publication and mention of the grant of the patent: **06.08.2025**
(21) Application number: 16909489.3
(22) Date of filing: 19.07.2016
(51) Int. Cl.: A61K 41/00, A61K 33/00, A61P 35/00, B01J 12/00, B01J 19/08, B01J 37/34

(54) **ANTITUMOR AQUEOUS SOLUTION MANUFACTURING DEVICE**
VORRICHTUNG ZUR HERSTELLUNG EINER ANTITUMORALEN WÄSSRIGEN LÖSUNG
DISPOSITIF DE FABRICATION D'UNE SOLUTION AQUEUSE ANTITUMORALE

(43) Date of publication of application: 29.05.2019
(73) Proprietor: Fuji Corporation, Chiryu-shi, Aichi 472-8686 (JP)
(72) Inventor: IKEDO, Toshiyuki, Chiryu, Aichi, (JP); NIWA, Tomoko, Chiryu, Aichi, (JP)
(74) Representative: Grünecker Patent- und Rechtsanwälte PartG mbB
(86) International application number: PCT/JP2016/071192
(87) International publication number: WO 2018/016014

(56) References cited:
- WO-A1-2016/035339
- WO-A1-2017/037775
- JP-A- 2014 113 517
- JP-A- 2014 113 517
- JP-A- 2014 189 441
- US-A1- 2015 030 693
- D. DOBRIN ET AL: "Degradation of methylparaben in water by corona plasma coupled with ozonation", ENVIRONMENTAL SCIENCE AND POLLUTION RESEARCH INTERNATIONAL, vol. 21, no. 21, 8 May 2014 (2014-05-08), DE, pages 12190 - 12197, XP055670485, ISSN: 0944-1344, DOI: 10.1007/s11356-014-2964-y
- KURAKE NAOYUKI ET AL: "Cell survival of glioblastoma grown in medium containing hydrogen peroxide and/or nitrite, or in plasma-activated medium", ARCHIVES OF BIOCHEMISTRY AND BIOPHYSICS, ACADEMIC PRESS, US, vol. 605, 26 January 2016 (2016-01-26), pages 102 - 108, XP029664809, ISSN: 0003-9861, DOI: 10.1016/J.ABB.2016.01.011
- KOSUKE SAITO ET AL: "Tumor-selective mitochondrial network collapse induced by atmospheric gas plasma-activated medium", ONCOTARGET, vol. 7, no. 15, 12 April 2016 (2016-04-12), United States, pages 19910 - 19927, XP055566684, ISSN: 1949-2553, DOI: 10.18632/oncotarget.7889
- HIROSHI KUWAHATA ET AL: "Generation of H2O2 in Distilled Water Irradiated with Atmospheric-Pressure Plasma Jet", E-JOURNAL OF SURFACE SCIENCE AND NANOTECHNOLOGY, 26 October 2013 (2013-10-26), pages 113 - 115, XP055657241, Retrieved from the Internet <URL:https://www.jstage.jst.go.jp/article/ejssnt/11/0/11_113/_pdf> [retrieved on 20131026], DOI: 10.1380/ejssnt.2013.113
- KURAKE N. ET AL.: "Cell survival of glioblastoma grown in medium containing hydrogen peroxide and/or nitrite, or in plasma-activated medium", ARCH. BIOCHEM. BIOPHYS., vol. 605, 26 January 2016 (2016-01-26), pages 102 - 108, XP029664809
- SAITO K. ET AL.: "Tumor-selective mitochondrial network collapse induced by atmospheric gas plasma-activated medium", ONCOTARGET, vol. 7, no. 15, 3 March 2016 (2016-03-03), pages 19910 - 19927, XP055566684
- UTSUMI F. ET AL.: "Variable susceptibility of ovarian cancer cells to non-thermal plasma- activated medium", ONCOLOGY REPORTS, vol. 35, 1 April 2016 (2016-04-01), pages 3169 - 3177, XP055566696

## Description

### Technical Field

The present invention relates to an antitumor aqueous solution manufacturing device suitable for manufacturing an antitumor aqueous solution capable of killing tumor cells.

### Background Art

Plasma, which uses an effect of excited electrons, has been applied in various ways to fields of electricity, chemistry, materials, and medical care. A technology has been reported that, as application of plasma to medical care, a solution manufactured through plasma irradiation has an effect of killing a tumor (PTL 1).
Further related background art is disclosed in PTL 2 to PTL 6.

### Citation List

### Patent Literature

PTL 1: International Publication No. 2013/128905
PTL2: Japanese Unexamined Patent Application Publication No. 2014-113517
PTL 3: WO 2016/035339 A1
PTL 4: US 2015/030693 A1
PTL 5: JP 2014 189441 A
PTL 6: WO 2017/037775 A1

### Summary of Invention

### Technical Problem

The effect of an aqueous solution having antitumor properties (hereinafter, referred to as "antitumor aqueous solution") that is generated through plasma irradiation varies in accordance with conditions such as a plasma irradiation time, and appropriate plasma irradiation conditions depend on the type of tumor.

With the plasma irradiation device of the related art, it is difficult to determine whether an appropriate antitumor effect is attained and it is difficult to select appropriate plasma irradiation conditions.

The invention is made in light of the situations, and a problem to be addressed is to provide an antitumor aqueous solution manufacturing device capable of manufacturing an antitumor aqueous solution having an appropriate antitumor effect.

### Solution to Problem

An antitumor aqueous solution manufacturing device of the invention that addresses the above-described problem is defined in claim 1.

Although an active component for an antitumor effect provided by an antitumor aqueous solution has not been adequately revealed, research findings tell that the degree of antitumor effect correlates with the hydrogen peroxide concentration. By controlling the hydrogen peroxide concentration instead of controlling the antitumor effect based on the findings, an antitumor aqueous solution that provides an appropriate effect depending on the type of tumor can be manufactured. For the appropriate hydrogen peroxide concentrations, desirable hydrogen peroxide concentrations can be preparatorily obtained depending on the type of tumor.

PTL 2 discloses a water quality control device that decomposes organic substances in water to be processed by using hydrogen peroxide. The device disclosed in PTL 2 has a technology that controls electric power to be supplied to plasma electrodes, which are used for plasma irradiation, based on the hydrogen peroxide concentration in the water to be processed after plasma irradiation and that controls the distance between the plasma electrodes such that the irradiation can be continued under certain conditions even when a plasma generating device changes with time (for example, a change in the distance between the plasma electrodes due to wear of the plasma electrodes). That is, in PTL 2, the technology controls the output and the distance between the plasma electrodes in accordance with a change with time of the plasma electrodes to make the effect through plasma irradiation constant; however, the invention of this application is a technology different from the technology of PTL 2 in that conditions such as a plasma irradiation time is changed to make the effect of an antitumor aqueous solution to be generated constant.

### Advantageous Effects of Invention

With the invention, by controlling the hydrogen peroxide concentration that correlates with the active component concentration instead of directly controlling the active component concentration through plasma irradiation, the manufacturing device capable of manufacturing an antitumor aqueous solution having an appropriate active component concentration depending on the tumor to which the antitumor aqueous solution is applied can be provided.

### Brief Description of Drawings

[Fig. 1] Fig. 1 is a perspective view of an antitumor aqueous solution manufacturing device (atmospheric-pressure plasma irradiation device).
[Fig. 2] Fig. 2 is an exploded view of a plasma generating device.
[Fig. 3] Fig. 3 is an exploded view of the plasma generating device.
[Fig. 4] Fig. 4 is a sectional view of the plasma generating device.
[Fig. 5] Fig. 5 is a perspective view of an antitumor aqueous solution manufacturing device.
[Fig. 6] Fig. 6 is a side view of the antitumor aqueous solution manufacturing device.
[Fig. 7] Fig. 7 is a side view of the antitumor aqueous solution manufacturing device.
[Fig. 8] Fig. 8 is a perspective view of the antitumor aqueous solution manufacturing device.
[Fig. 9] Fig. 9 is a schematic diagram of a cooling device.
[Fig. 10] Fig. 10 is a block diagram of a control device.

### Description of Embodiments

An antitumor aqueous solution manufacturing device of the invention is described below in detail with reference to the drawings for example. An antitumor aqueous solution manufacturing device of this embodiment manufactures an antitumor aqueous solution by irradiating a raw material solution with plasma and thereby generating an antitumor component in the raw material solution. The antitumor aqueous solution manufacturing device of this embodiment is implemented by atmospheric-pressure plasma irradiation device 10 described below that performs plasma irradiation under the atmospheric pressure.

Plasma irradiation is performed while the hydrogen peroxide concentration in the raw material solution is measured, and plasma irradiation is ended when the measured concentration has fallen in a predetermined concentration range. Because the hydrogen peroxide concentration correlates with the plasma irradiation conditions, controlling the hydrogen peroxide concentration provides an antitumor aqueous solution appropriately irradiated with plasma. The predetermined concentration range has appropriate values that vary in accordance with, for example, the type of tumor to which the antitumor aqueous solution is applied. The raw material solution during plasma irradiation may be left in a stationary state or in a circulation state. When plasma irradiation is performed, the raw material solution may be irradiated with plasma from above the solution surface.

The manufactured antitumor aqueous solution can affect a tumor existing in a living body by being directly administered to the living body. The administration of the antitumor aqueous solution to the living body can employ a method of bringing the antitumor aqueous solution into contact with the tumor in a state exposed by a surgical treatment. For example, when a tumor was attempted to be removed but was not completely removed by a surgical treatment, the remaining tumor can be killed by the contact between the antitumor aqueous solution and the tumor through washing or the like during the surgical treatment.

The raw material solution is an aqueous solution. For example, the raw material solution is desirably an aqueous solution prepared by adding a solute containing at least one component selected from disodium hydrogen phosphate (Na₂HPO₄), sodium hydrogen carbonate (NaHCO₃), L-glutamine, L-histidine, and L-tyrosine disodium (L-tyrosine·2Na) to water. The raw material solution using the aqueous solution is irradiated with plasma and thus a highly effective antitumor aqueous solution with can be obtained. The raw material solution is desirably a living body fluid, or a culture solution for living body cells.

### Configuration of Atmospheric-pressure Plasma Irradiation Device

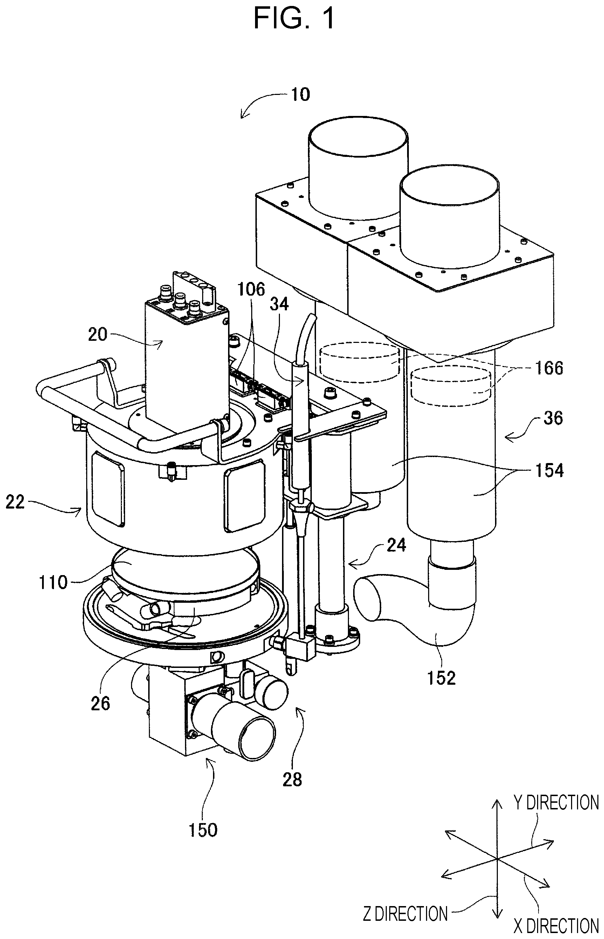

Fig. 1 illustrates atmospheric-pressure plasma irradiation device 10 of an embodiment of the invention. Atmospheric-pressure plasma irradiation device 10 irradiates a culture solution serving as a raw material solution with plasma under the atmospheric pressure. The culture solution is held in petri dish 110 in a stationary state.

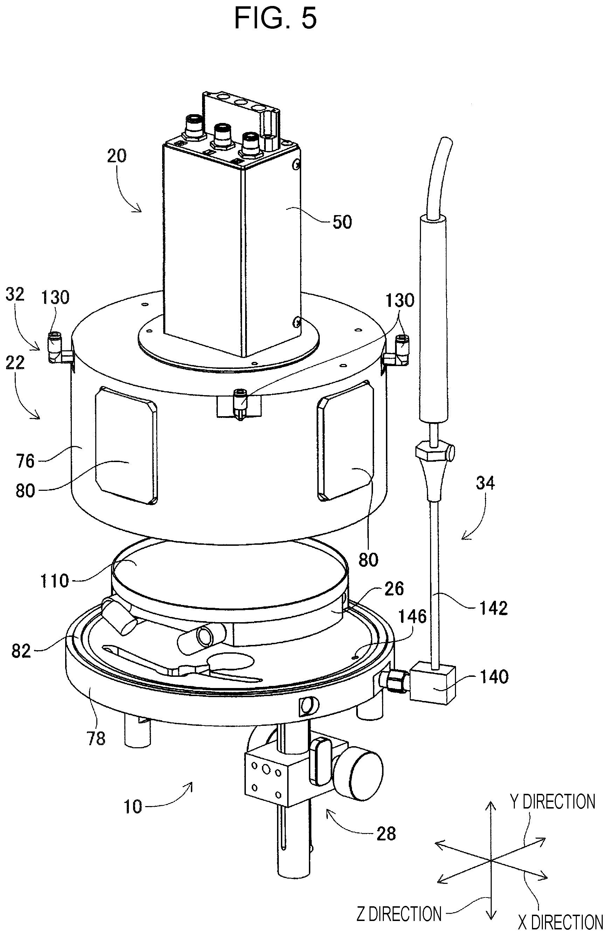

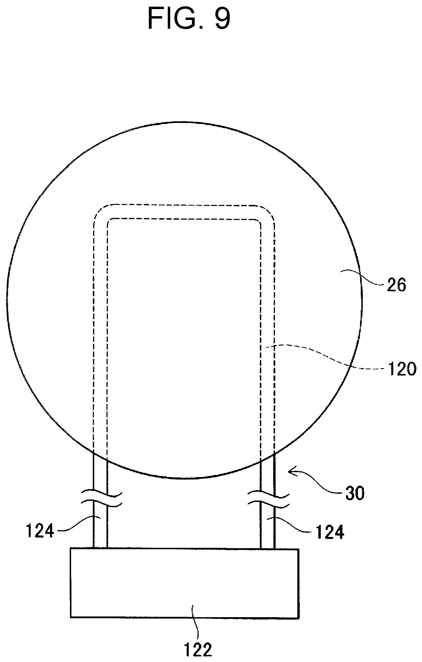

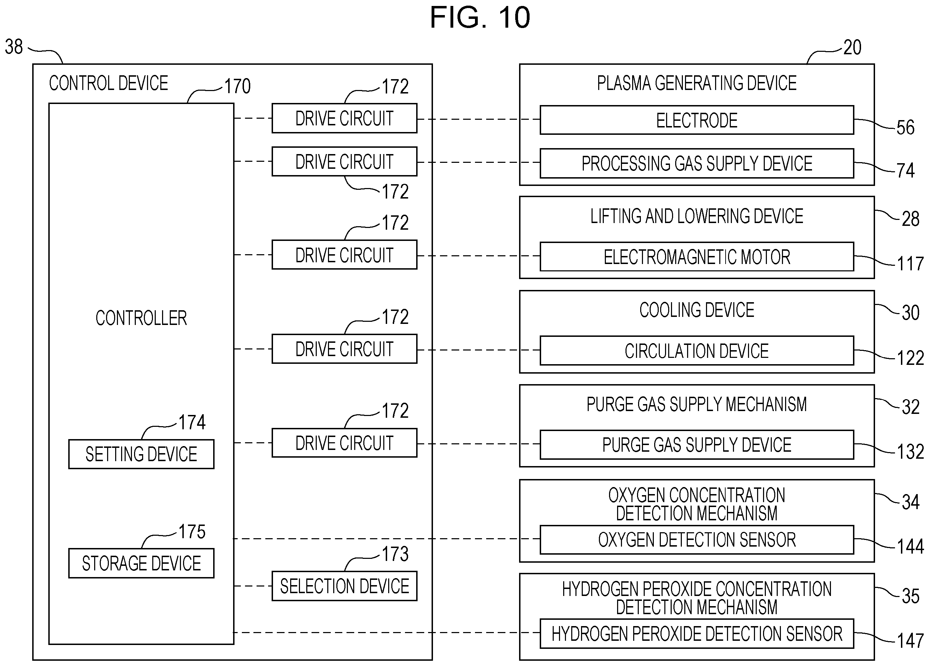

Atmospheric-pressure plasma irradiation device 10 includes plasma generating device 20, cover housing 22, opening and closing mechanism 24, stage 26 serving as a holding portion that holds the raw material solution in petri dish 110, lifting and lowering device 28, cooling device (see Fig. 9) 30, purge gas supply mechanism (see Fig. 5) 32, oxygen concentration detection mechanism 34, hydrogen peroxide concentration detection mechanism (see Fig. 10) 35, exhaust mechanism 36, and control device (see Fig. 10) 38. Note that a width direction of atmospheric-pressure plasma irradiation device 10 is referred to as X direction, a depth direction of atmospheric-pressure plasma irradiation device 10 is referred to as Y direction, and a direction perpendicular to the X and Y directions, that is, an up-down direction, is referred to as Z direction.

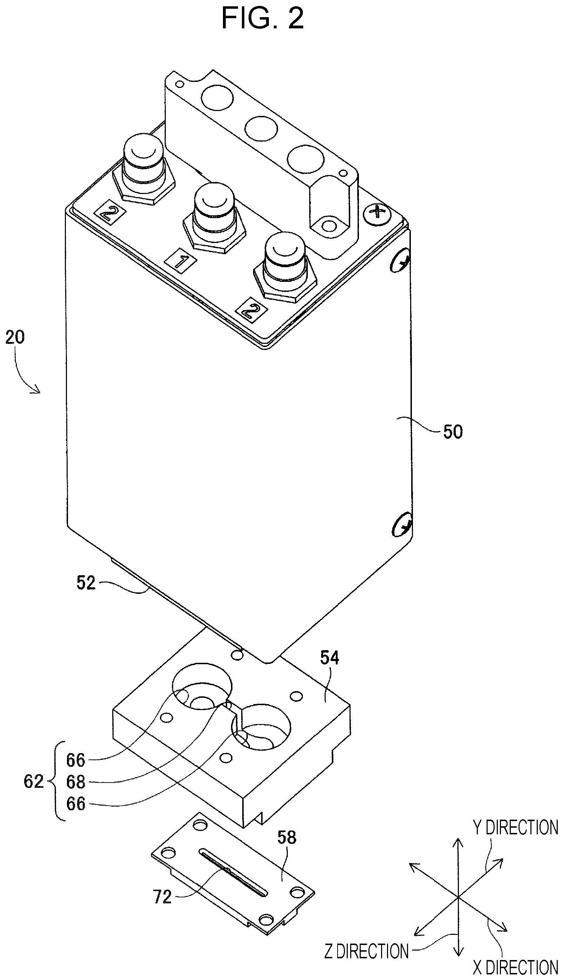

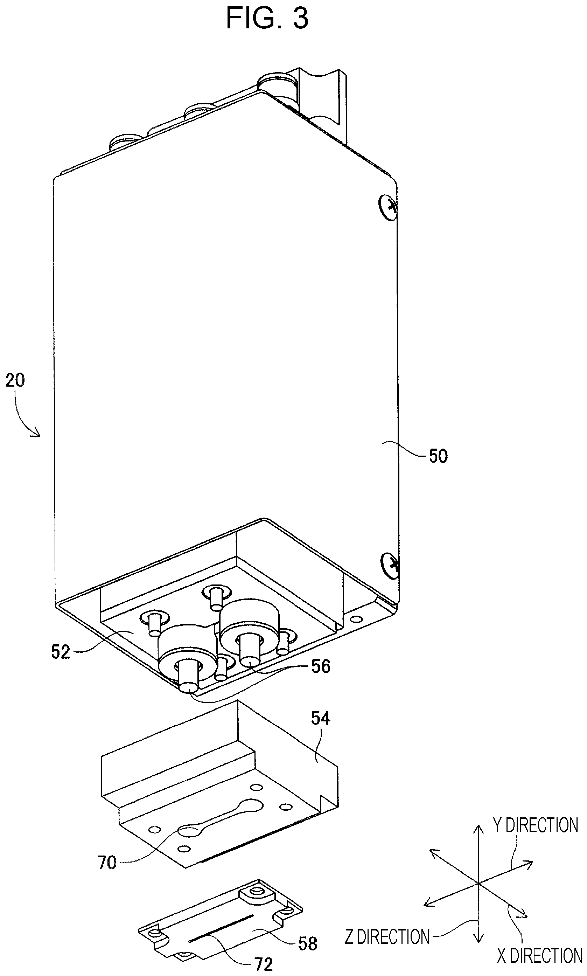

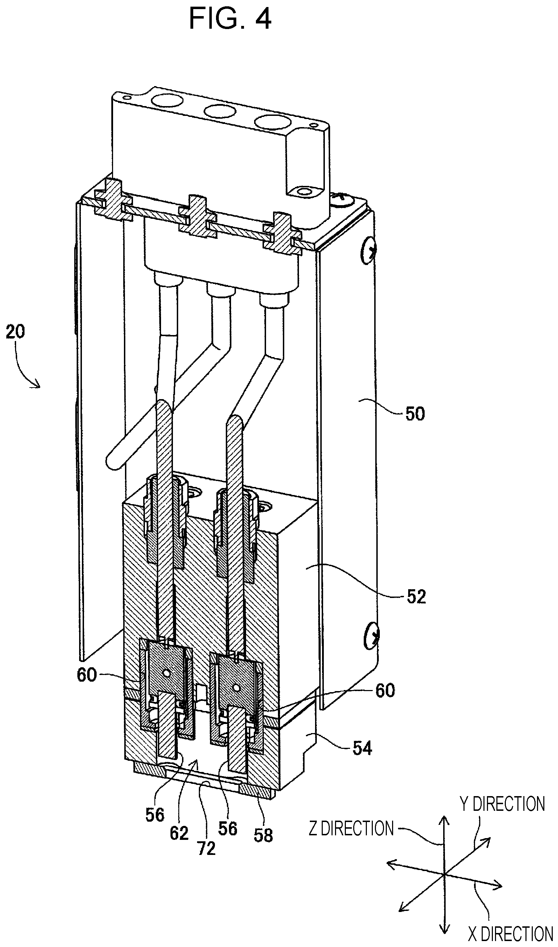

As illustrated in Figs. 2 to 4, plasma generating device 20 includes cover 50, upper block 52, lower block 54, one pair of electrodes 56, and nozzle block 58. Cover 50 substantially has a lidded rectangular tubular shape, and upper block 52 is arranged in cover 50. Upper block 52 is substantially rectangular-parallelepiped and is formed of a ceramic. One pair of columnar recessed portions 60 are formed in a lower surface of upper block 52.

Lower block 54 is substantially rectangular-parallelepiped and is formed of a ceramic. Recessed portion 62 is formed in an upper surface of lower block 54, and recessed portion 62 is configured of one pair of columnar recessed portions 66 and connecting recessed portion 68 that connects one pair of columnar recessed portions 66 to each other. Lower block 54 is fixed to the lower surface of upper block 52 in a state in which lower block 54 protrudes from a lower end of cover 50, and columnar recessed portions 60 of upper block 52 communicate with columnar recessed portions 66 of lower block 54. Columnar recessed portions 60 and columnar recessed portions 66 have substantially the same diameter. A bottom surface of recessed portion 62 has slit 70 that extends through lower block 54 to a lower surface thereof.

One pair of electrodes 56 are arranged in columnar spaces defined by columnar recessed portions 60 of upper block 52 and columnar recessed portions 66 of lower block 54. Electrodes 56 have an outer diameter smaller than the inner diameter of columnar recessed portions 60, 66. Nozzle block 58 has a substantially flat-plate shape and is fixed to the lower surface of lower block 54. Nozzle block 58 has emission port 72 that communicates with slit 70 of lower block 54, and emission port 72 extends through nozzle block 58 in the up-down direction.

Plasma generating device 20 further has processing gas supply device (see Fig. 10) 74. Processing gas supply device 74 supplies a processing gas in which an active gas, such as oxygen or hydrogen, is mixed with an inert gas, such as argon or nitrogen, at a given ratio; and processing gas supply device 74 is connected to the columnar spaces defined by columnar recessed portions 60, 66 and an upper area of connecting recessed portion 68 via a pipe (not illustrated). Thus, the processing gas is supplied into recessed portion 62 from the gaps between electrodes 56 and columnar recessed portions 66 and from the upper area of connecting recessed portion 68.

With the structure, plasma generating device 20 emits plasma from emission port 72 of nozzle block 58. In detail, the processing gas is supplied into recessed portion 62 by processing gas supply device 74. In this case, a voltage is applied to one pair of electrodes 56 and current flows between one pair of electrodes 56 in recessed portion 62. Thus, an electrical discharge occurs between one pair of electrodes 56, and the processing gas is plasmarized by the electrical discharge. The plasma is emitted from emission port 72 via slit 70.

As illustrated in Fig. 5, cover housing 22 includes upper cover 76 and lower cover 78. Upper cover 76 substantially has a lidded cylindrical shape, and a lid of upper cover 76 has a through-hole (not illustrated) with a shape corresponding to lower block 54 of plasma generating device 20. Cover 50 of plasma generating device 20 is fixed in a standing state on the lid of upper cover 76 so as to cover the through-hole. Thus, lower block 54 and nozzle block 58 of plasma generating device 20 protrude toward the inside of upper cover 76 so as to extend in the Z direction. Accordingly, plasma generated by plasma generating device 20 is emitted in the Z direction from emission port 72 of nozzle block 58 toward the inside of upper cover 76.

Moreover, a side surface of upper cover 76 has three substantially rectangular through-holes (not illustrated) formed at three equally spaced positions, and transparent glass plates 80 are arranged so as to close the through-holes. Accordingly, the inside of upper cover 76 can be visually checked via glass plates 80.

Lower cover 78 of cover housing 22 is substantially disk-shaped and is fixed to a casing (not illustrated) of a placing portion on which atmospheric-pressure plasma irradiation device 10 is placed. Lower cover 78 has a larger outer diameter than the outer diameter of upper cover 76, and annular gasket 82 with the same diameter as the diameter of upper cover 76 is arranged on an upper surface of lower cover 78. When upper cover 76 slides downward by opening and closing mechanism 24, upper cover 76 comes into close contact with gasket 82, and the inside of cover housing 22 is hermetically sealed.

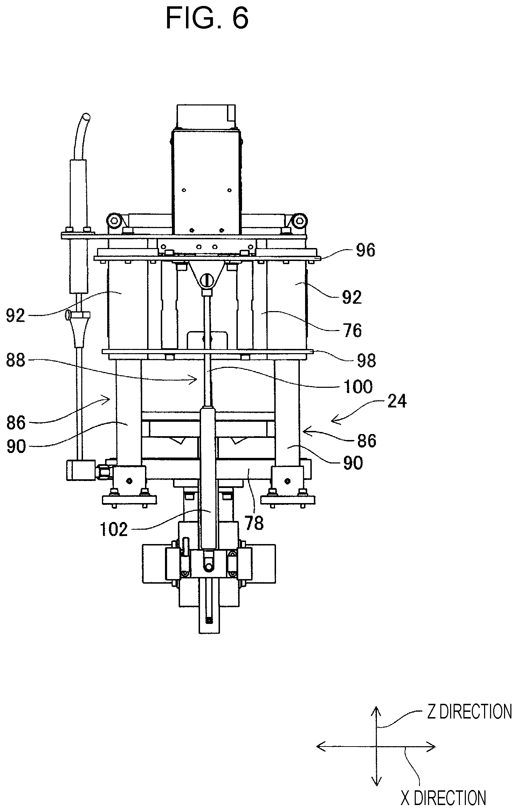

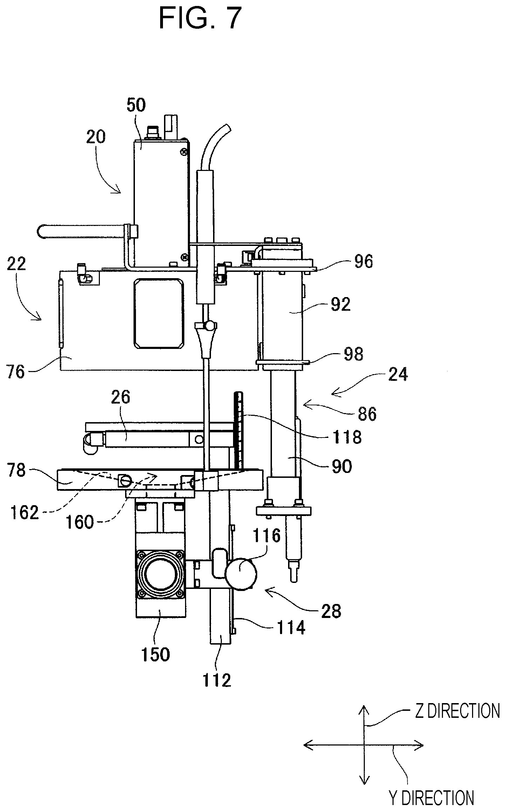

In detail, as illustrated in Figs. 6 and 7, opening and closing mechanism 24 includes one pair of slide mechanisms 86 and air cylinder 88. Each of slide mechanisms 86 includes support shaft 90 and slider 92. Support shaft 90 stands on the casing of the placing portion so as to extend in the Z direction. Moreover, slider 92 is substantially cylindrical, and is fitted onto support shaft 90 so as to be slidable in the axial direction of support shaft 90.

Upper cover 76 is held at slider 92 by using upper bracket 96 and lower bracket 98. Thus, upper cover 76 is slidable in the Z direction, that is, the up-down direction.

Air cylinder 88 includes rod 100, a piston (not illustrated), and cylinder 102. Rod 100 is arranged to extend in the Z direction, and an upper end portion of rod 100 is fixed to upper cover 76. Moreover, a lower end portion of rod 100 is fixed to the piston. The piston is fitted into cylinder 102 from an upper end of cylinder 102, and slidably moves in cylinder 102. Cylinder 102 is fixed to the casing of the placing portion at the lower end portion, and a predetermined amount of air is sealed in cylinder 102.

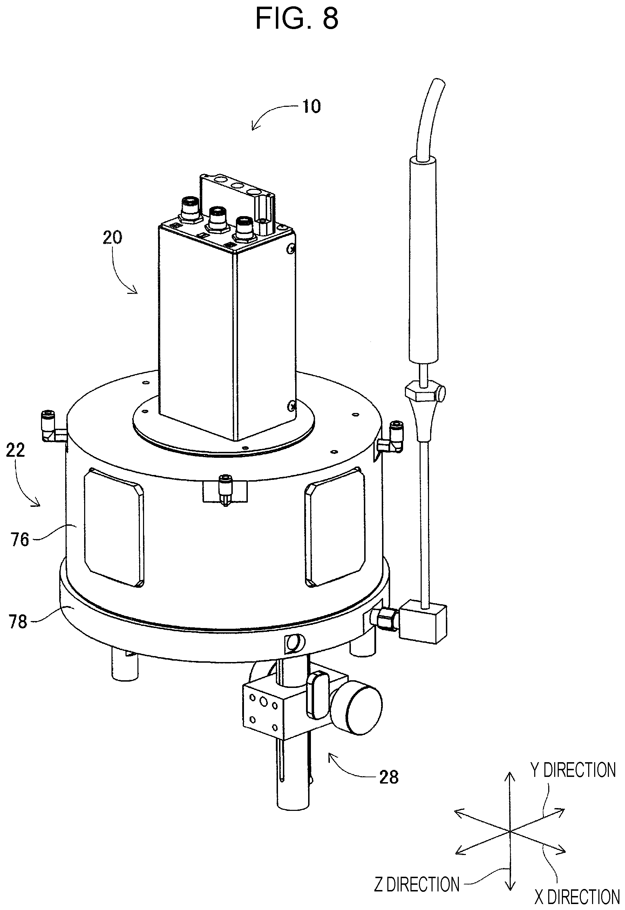

Thus, air cylinder 88 functions as a damper, and upper cover 76 is prevented from being rapidly lowered. The air pressure in cylinder 102 is a pressure that can be compressed by the weight of an integrated body that slides together with upper cover 76, or more specifically, the weights of upper cover 76, plasma generating device 20, slider 92, and another member. That is, when an operator releases upper cover 76 while upper cover 76 is lifted, upper cover 76 is lowered by the weight of upper cover 76 and another member. Upper cover 76 comes into close contact with gasket 82 of lower cover 78, and as illustrated in Fig. 8, the inside of cover housing 22 is hermetically sealed by upper cover 76 and lower cover 78.

When the operator lifts upper cover 76, the inside of cover housing 22 is opened. Magnets (see Fig. 1) 106 are fixed to an upper surface of upper cover 76, and when upper cover 76 is lifted, magnets 106 stick to the casing of the placing portion. When magnets 106 stick to the casing of the placing portion, the lifted state of upper cover 76, that is, the open state of cover housing 22 is maintained.

Stage 26 is substantially disk-shaped, and petri dish 110 is placed on an upper surface of stage 26. One or two hydrogen peroxide detection sensors (see Fig. 10, not illustrated in the other drawings) 147 that are connected to hydrogen peroxide concentration detection mechanism 35 are arranged such that hydrogen peroxide detection sensors 147 can be inserted into petri dish 110. Stage 26 has a smaller outer diameter than the outer diameter of lower cover 78. Stage 26 is arranged on the upper surface of lower cover 78.

As illustrated in Fig. 7, lifting and lowering device 28 includes support rod 112, rack 114, pinion 116, and electromagnetic motor (see Fig. 10) 117. Lower cover 78 has a through-hole (not illustrated) that extends therethough in the up-down direction, and support rod 112 is inserted through the through-hole. Support rod 112 has a smaller outer diameter than the inner diameter of the through-hole, and support rod 112 is movable in the up-down direction, that is, in the Z direction. The lower surface of stage 26 is fixed to an upper end of support rod 112.

Rack 114 is fixed to an outer peripheral surface of a portion of support rod 112 extending downward from lower cover 78 to extend in the axial direction of support rod 112. Pinion 116 meshes with rack 114, and is rotated by driving of electromagnetic motor 117. Pinion 116 is rotatably held by the casing of the placing portion. With the structure, when pinion 116 is rotated by driving of electromagnetic motor 117, support rod 112 moves in the Z direction, and stage 26 is lifted and lowered. Gaging rod 118 stands on the upper surface of lower cover 78, at a position next to stage 26. An outer peripheral surface of gaging rod 118 has a scale, and the height of stage 26 in the Z direction, that is, the lifted and lowered amount of stage 26 can be visually checked by using the scale.

As illustrated in Fig. 9, cooling device 30 includes cooling water channel 120, circulation device 122, and pipe 124. Cooling water channel 120 extends through the inside of stage 26 in the plane direction of stage 26 to have an "angular C" shape, and is open at two positions of the outer peripheral surface of stage 26. Circulation device 122 cools water and circulates the cooled water. Circulation device 122 is connected to cooling water channel 120 via pipe 124. Thus, when cooling water flows in stage 26, stage 26 is cooled.

As illustrated in Fig. 5, purge gas supply mechanism 32 includes four air joints (in the drawing, three air joints are illustrated) 130 and purge gas supply mechanism (see Fig. 10) 132. Four air joints 130 are provided at four equally spaced positions at an upper end portion of a side surface of upper cover 76, and air joints 130 are open to the inside of upper cover 76. Purge gas supply device 132 supplies an inert gas such as nitrogen, and is connected to air joints 130 via pipes (not illustrated). With the structure, purge gas supply mechanism 32 supplies the inert gas such as argon into upper cover 76.

Oxygen concentration detection mechanism 34 includes air joint 140, pipe 142, and oxygen detection sensor (see Fig. 10) 144. Lower cover 78 has a through-hole (not illustrated) that extends from the upper surface to the side surface of lower cover 78. Opening 146 of the through-hole at the upper surface of lower cover 78 is positioned inside gasket 82. In contrast, air joint 140 is connected to an opening of the through-hole at the side surface of lower cover 78. Oxygen detection sensor 144 detects the oxygen concentration, and is connected to air joint 140 via pipe 142. With the structure, oxygen concentration detection mechanism 34 detects the oxygen concentration in cover housing 22 when cover housing 22 is hermetically sealed.

As illustrated in Fig. 1, exhaust mechanism 36 includes L-shaped pipe 150, connecting pipe 152, and main pipe 154. As illustrated in Fig. 7, lower cover 78 has duct port 160 that is open to the upper surface and a lower surface of lower cover 78. The opening of duct port 160 at the upper surface of lower cover 78 defines tapered surface 162 having an inner diameter that increases upward. That is, when cover housing 22 is hermetically sealed, tapered surface 162 is inclined toward an inner wall surface of upper cover 76. In contrast, L-shaped pipe 150 is connected to the opening of duct port 160 at the lower surface of lower cover 78. Main pipe 154 is connected to L-shaped pipe 150 via connecting pipe 152. Note that a portion of connecting pipe 152 near L-shaped pipe 150 is not illustrated. Moreover, ozone filter 166 is arranged in main pipe 154. Ozone filter 166 is formed of activated carbon and sucks ozone.

As illustrated in Fig. 10, control device 38 includes controller 170, multiple drive circuits 172, and selection device 173. Multiple drive circuits 172 are connected to electrodes 56, processing gas supply device 74, electromagnetic motor 117, circulation device 122, and purge gas supply device 132. Controller 170 includes a CPU, ROM, RAM, and the like, is mainly composed of a computer, and is connected to multiple drive circuits 172. Thus, controller 170 controls operations of plasma generating device 20, lifting and lowering device 28, cooling device 30, and purge gas supply mechanism 32. Moreover, controller 170 is connected to oxygen detection sensor 144, and hydrogen peroxide detection sensor 147 of hydrogen peroxide concentration detection mechanism 35. Thus, controller 170 acquires detection results of oxygen detection sensor 144 and hydrogen peroxide detection sensor 147, that is, the oxygen concentration in cover housing 22 and the hydrogen peroxide concentration in the raw material solution.

The RAM and ROM of controller 170 function as storage device 175, and store values of appropriate hydrogen peroxide concentrations that are set for each of multiple purposes of use of antitumor aqueous solutions to be manufactured. The appropriate hydrogen peroxide concentrations can be previously set by an experiment or the like.

The antitumor aqueous solution that is manufactured by plasma irradiation device 10 of this embodiment has appropriate plasma irradiation conditions depending on the type of tumor to which the antitumor aqueous solution is applied, and the plasma irradiation conditions are handled as values of hydrogen peroxide concentrations. Selection device 173 that is connected to control device 38 selects the tumor to which the antitumor aqueous solution to be manufactured is applied.

In controller 170, setting device 174 installed as a logic extracts values of corresponding hydrogen peroxide concentrations stored in the storage device in accordance with the type of tumor selected by selection device 173, and sets the values as a predetermined range of the values of hydrogen peroxide concentrations that are used for control in a final phase of plasma irradiation. Although not particularly limited, an example of selection device 173 may be a keyboard, a mouse, a touch panel, or any type of switch that is generally used for a computer. Controller 170 performs plasma irradiation until the value of hydrogen peroxide concentration in the raw material solution falls within the predetermined range that has been set. As described above, a user can set appropriate hydrogen peroxide concentrations simply by selecting a type of tumor of an application target by using selection device 173 without inputting appropriate hydrogen peroxide concentrations, and can manufacture an antitumor aqueous solution that is manufactured through plasma irradiation under appropriate conditions.

### Plasma Irradiation Using Atmospheric-pressure Plasma Irradiation Device

By irradiating a raw material solution with plasma, the raw material solution is activated and an antitumor aqueous solution is obtained. Plasma irradiation is performed until the hydrogen peroxide concentration in the culture solution subjected to plasma irradiation falls within an appropriate range. For the appropriate range of hydrogen peroxide concentrations, desirable hydrogen peroxide concentrations can be preparatorily obtained depending on the type of tumor to which the antitumor aqueous solution is applied. The values of hydrogen peroxide concentrations are desirably values obtained through plasma irradiation that has been started from certain initial conditions. By setting the consistent initial conditions, the correlation between the antitumor component concentration and the hydrogen peroxide concentration in the antitumor aqueous solution is possibly enhanced.

With atmospheric-pressure plasma irradiation device 10, using the configuration described above, by placing petri dish 110 storing the culture solution on stage 26 and hermetically sealing cover housing 22, the culture solution can be irradiated with plasma under predetermined conditions. Hereinafter, a method of irradiating the culture solution with plasma under the predetermined conditions is described below in detail.

Specifically, petri dish 110 storing the culture solution is placed on stage 26. Hydrogen peroxide detection sensor 147 is inserted into petri dish 110. Then, stage 26 is lifted or lowered to a desirable height by using lifting and lowering device 28. Thus, the distance between emission port 72 of plasma and the culture solution serving as a body to be irradiated with plasma can be desirably set. The lifted or lowered height of stage 26 can be checked by using the scale of gaging rod 118.

Then, upper cover 76 is lowered and cover housing 22 is hermetically sealed. Purge gas supply mechanism 32 supplies the inert gas into cover housing 22. At this time, oxygen concentration detection mechanism 34 detects the oxygen concentration in cover housing 22. After the detected oxygen concentration has been below a previously set threshold, plasma generating device 20 emits plasma into cover housing 22.

Before plasma irradiation is started, a target to which the antitumor aqueous to be manufactured is applied, and use conditions are selected by operating selection device 173. Setting device 174 of controller 170 sets a predetermined range of values of hydrogen peroxide concentrations as a target with reference to data in the storage device based on the selected purpose of use. Plasma irradiation is performed until the hydrogen peroxide concentration in petri dish 110 falls within the predetermined range that has been set. Setting device 174 can set, in addition to the predetermined range of the values of hydrogen peroxide concentration, other plasma irradiation conditions (for example, irradiation output, oxygen concentration, and temperature of raw material solution) in accordance with the purpose of use selected by selection device 173.

While plasma irradiation is performed, the inert gas is continuously supplied into cover housing 22. Supplying the inert gas into cover housing 22 exhausts the air in cover housing 22 to the outside of cover housing 22. In this case, the oxygen concentration in cover housing 22 is regulated, and the conditions that affect plasma irradiation are controlled. More specifically, since plasma contains active radicals, when the active radicals react with oxygen, the active radicals become ozone and decrease the effect of plasma irradiation. Regulating the oxygen concentration in cover housing 22 can control the influence of the oxygen concentration on the effect of the culture solution irradiated with plasma. In addition, the culture solution can be irradiated with plasma under the same conditions. Thus, the antitumor aqueous solution having a certain antitumor effect can be efficiently manufactured.

With atmospheric-pressure plasma irradiation device 10, as described above, the distance between emission port 72 of plasma and the culture solution is desirably set. Thus, the irradiation distance, which is as a plasma irradiation condition, can be optimized, and the antitumor aqueous solution having a certain antitumor effect can be efficiently manufactured.

The plasma generating device radiates plasma from above the solution surface of the culture solution in petri dish 110. Thus, plasma is radiated in a wide range and hence the processing speed is increased.

During plasma irradiation, cooling device 30 circulates cooling water in stage 26. Cooling stage 26 restricts a temperature rise of the culture solution in petri dish 110 by plasma irradiation, and prevents the culture solution from vaporizing. Controlling the temperature of the cooling water regulates the temperature of the culture solution during plasma irradiation.

Lower cover 78 has duct port 160. By this, when the inert gas is supplied into cover housing 22, the pressure in cover housing 22 becomes positive pressure, and the gas is naturally exhausted from the inside of cover housing 22. Moreover, duct port 160 of lower cover 78 has tapered surface 162 whose inner diameter increases toward the upper surface of lower cover 78. Thus, the exhaust of the gas from the inside of cover housing 22 can be promoted. Further, exhaust mechanism 36 is provided with ozone filter 166. Thus, even when plasma reacts with oxygen and ozone is generated, the exhaust of the ozone to the outside can be prevented.

### Industrial Applicability

The antitumor aqueous solution manufacturing device of the invention can manufacture an antitumor aqueous solution having an appropriate effect depending on the tumor to which the antitumor aqueous solution is applied. Reference Signs List

10: atmospheric-pressure plasma irradiation device
20: plasma generating device
22: cover housing
26: stage (holding portion)
28: lifting and lowering device
30: cooling device
35: hydrogen peroxide concentration detection mechanism
38: control device
72: emission port
132: purge gas supply device
144: oxygen detection sensor
147: hydrogen peroxide detection sensor

## Claims

1. An antitumor aqueous solution manufacturing device (10) configured to manufacture an antitumor aqueous solution having an antitumor effect, comprising:
a holding portion (26), in the form of a stage (26), configured to hold a raw material solution in which a raw material of the antitumor aqueous solution is dissolved, wherein the holding portion (26) is configured to hold the raw material solution in a stationary state or in a repetitive circulation state;
a plasma generating device (20) configured to generate plasma to be radiated on the raw material solution in the holding portion (26), wherein the plasma generating device (20) is configured to radiate plasma from above a solution surface of the raw material solution held in the holding portion (26);
a hydrogen peroxide detection sensor (147) configured to measure a hydrogen peroxide concentration in the raw material solution in the holding portion (26); and
a control device (38) configured to control the plasma generating device (20),
wherein the control device (38) is configured to control the plasma generating device (20) such that the hydrogen peroxide concentration measured by the hydrogen peroxide detection sensor (147) falls within a predetermined range.

2. The antitumor aqueous solution manufacturing device (10) according to Claim 1, wherein the raw material solution is an aqueous solution prepared by adding a solute containing at least one component selected from disodium hydrogen phosphate (Na₂HPO₄), sodium hydrogen carbonate (NaHCO₃), L-glutamine, L-histidine, and L-tyrosine disodium (L-tyrosine·2Na) to water.

3. The antitumor aqueous solution manufacturing device (10) according to Claim 1 or 2,
wherein the control device (38) includes
a storage device configured to store values of appropriate hydrogen peroxide concentrations that are set for each of multiple purposes of use of the antitumor aqueous solution,
a selection device (173) configured to select one of the multiple purposes of use, and
a setting device (174) configured to set the predetermined range based on the values of the appropriate hydrogen peroxide concentrations corresponding to the purpose of use selected by the selection device (173).

4. The antitumor aqueous solution manufacturing device (10) according to any one of Claims 1 to 3, wherein the raw material solution is a living body fluid, or a culture solution for living body cells.

## Patentansprüche

1. Eine Vorrichtung (10) zur Herstellung einer wässrigen Antitumorlösung, die so konfiguriert ist, dass sie eine wässrige Antitumorlösung mit einer Antitumorwirkung herstellt, aufweisend:
einen Halteabschnitt (26) in Form einer Stufe (26), der so konfiguriert ist, dass er eine Rohmateriallösung hält, in der ein Rohmaterial der wässrigen Antitumorlösung gelöst ist, wobei der Halteabschnitt (26) so konfiguriert ist, dass er die Rohmateriallösung in einem stationären Zustand oder in einem sich wiederholenden Umlaufzustand hält;
eine Plasmaerzeugungsvorrichtung (20), die so konfiguriert ist, dass sie Plasma erzeugt, das auf die Rohmateriallösung in dem Halteabschnitt (26) gestrahlt werden soll, wobei die Plasmaerzeugungsvorrichtung (20) so konfiguriert ist, dass sie Plasma von oberhalb einer Lösungsoberfläche der in dem Halteabschnitt (26) gehaltenen Rohmateriallösung strahlt;
einen Wasserstoffperoxid-Detektionssensor (147), der so konfiguriert ist, dass er eine Wasserstoffperoxidkonzentration in der Rohmateriallösung in dem Halteabschnitt (26) misst; und
eine Steuereinheit (38), die zur Steuerung der Plasmaerzeugungsvorrichtung (20) konfiguriert ist,
wobei die Steuereinheit (38) so konfiguriert ist, dass sie die Plasmaerzeugungsvorrichtung (20) so steuert, dass die von dem Wasserstoffperoxid-Detektionssensor (147) gemessene Wasserstoffperoxidkonzentration in einen vorgegebenen Bereich fällt.

2. Die Vorrichtung zur Herstellung einer wässrigen Antitumorlösung (10) nach Anspruch 1, wobei die Rohmateriallösung eine wässrige Lösung ist, die durch Zugabe eines gelösten Stoffes, der mindestens eine Komponente, ausgewählt aus Dinatriumhydrogenphosphat (Na₂HPO₄), Natriumhydrogencarbonat (NaHCO₃), L-Glutamin, L-Histidin und L-Tyrosindinatrium (L-Tyrosin×2Na) enthält, zu Wasser hergestellt wird.

3. Die Vorrichtung (10) zur Herstellung einer wässrigen Antitumorlösung nach Anspruch 1 oder 2,
wobei die Steuereinheit (38) umfasst
eine Speichervorrichtung, die so konfiguriert ist, dass sie Werte geeigneter WasserstoffperoxidKonzentrationen speichert, die für jeden von mehreren Verwendungszwecken der wässrigen Antitumorlösung eingestellt werden,
eine Auswahlvorrichtung (173), die so konfiguriert ist, dass sie einen der mehreren Verwendungszwecke auswählt, und
eine Einstellvorrichtung (174), die so konfiguriert ist, dass sie den vorbestimmten Bereich auf der Grundlage der Werte der geeigneten Wasserstoffperoxidkonzentrationen entsprechend dem von der Auswahlvorrichtung (173) ausgewählten Verwendungszweck einstellt.

4. Die Vorrichtung (10) zur Herstellung einer wässrigen Antitumorlösung nach einem der Ansprüche 1 bis 3, wobei die Rohmateriallösung eine lebende Körperflüssigkeit oder eine Kulturlösung für lebende Körperzellen ist.

## Revendications

1. Dispositif de fabrication de solution aqueuse antitumorale (10) configuré pour fabriquer une solution aqueuse antitumorale ayant un effet antitumoral, comprenant :
une partie de maintien (26), sous la forme d'un étage (26), configurée pour maintenir une solution de matière première dans laquelle une matière première de la solution aqueuse antitumorale est dissoute, dans lequel la partie de maintien (26) est configurée pour maintenir la solution de matière première dans un état stationnaire ou dans un état de circulation répétitive ;
un dispositif de génération de plasma (20) configuré pour générer du plasma à rayonner sur la solution de matière première dans la partie de maintien (26), dans lequel le dispositif de génération de plasma (20) est configuré pour rayonner du plasma à partir d'une surface de solution de la solution de matière première maintenue dans la partie de maintien (26) ;
un capteur de détection de peroxyde d'hydrogène (147) configuré pour mesurer une concentration de peroxyde d'hydrogène dans la solution de matière première dans la partie de maintien (26) ; et
un dispositif de commande (38) configuré pour commander le dispositif de génération de plasma (20),
dans lequel le dispositif de commande (38) est configuré pour commander le dispositif de génération de plasma (20) de sorte que la concentration de peroxyde d'hydrogène mesurée par le capteur de détection de peroxyde d'hydrogène (147) tombe dans une plage prédéterminée.

2. Dispositif de fabrication de solution aqueuse antitumorale (10) selon la revendication 1, dans lequel la solution de matière première est une solution aqueuse préparée en ajoutant à l'eau un soluté contenant au moins un composant sélectionné parmi l'hydrogénophosphate disodique (Na₂HPO₄), l'hydrogénocarbonate de sodium (NaHCO₃), la L-glutamine, la L-histidine, et la L-tyrosine disodique (L-tyrosine-2Na).

3. Dispositif de fabrication de solution aqueuse antitumorale (10) selon la revendication 1 ou 2, dans lequel le dispositif de commande (38) inclut
un dispositif de stockage configuré pour stocker des valeurs de concentrations appropriées en peroxyde d'hydrogène qui sont définies pour chacun des plusieurs objectifs d'utilisation de la solution aqueuse antitumorale,
un dispositif de sélection (173) configuré pour sélectionner l'un des plusieurs objectifs d'utilisation, et
un dispositif de réglage (174) configuré pour régler la plage prédéterminée sur la base des valeurs des concentrations appropriées de peroxyde d'hydrogène correspondant à l'objectif d'utilisation sélectionné par le dispositif de sélection (173).

4. Dispositif de fabrication de solution aqueuse antitumorale (10) selon l'une quelconque des revendications 1 à 3, dans lequel la solution de matière première est un fluide de corps vivant, ou une solution de culture de cellules de corps vivant.
